# EUROPEAN PATENT SPECIFICATION

(11) **EP 4 273 208 B1**
(45) Date of publication and mention of the grant of the patent: **04.06.2025**
(21) Application number: 23168124.8
(22) Date of filing: 14.04.2023
(51) Int. Cl.: C10L 10/08, B82Y 20/00, C01B 32/15, C09K 11/65, C10L 1/00

(54) **PROCESS OF PREPARATION OF HYDROPHOBIC FLUORESCENT CARBON QUANTUM DOT FOR MARKER APPLICATION IN HYDROCARBON LIQUIDS**
VERFAHREN ZUR HERSTELLUNG VON HYDROPHOBEN FLUORESZIERENDEN KOHLENSTOFFQUANTENPUNKTEN ZUR MARKERANWENDUNG IN KOHLENWASSERSTOFFFLÜSSIGKEITEN
PROCÉDÉ DE PRÉPARATION DE POINT QUANTIQUE DE CARBONE FLUORESCENT HYDROPHOBE POUR APPLICATION DE MARQUEUR DANS DES LIQUIDES HYDROCARBONÉS

(30) Priority: 04.05.2022 IN 202221025921
(43) Date of publication of application: 08.11.2023
(73) Proprietor: Indian Oil Corporation Limited, Bandra (East) Maharashtra, Mumbai 400 051 (IN)
(72) Inventor: OTA, Jyotiranjan, 121007 Faridabad (IN); SINHA, Arjyabaran, 121007 Faridabad (IN); MESHRAM, Rahul, 121007 Faridabad (IN); HAIT, Samik Kumar, 121007 Faridabad (IN); CHANDRASEKARAN, Kannan, 121007 Faridabad (IN); RAMAKUMAR, Sankara Sri Venkata, 121007 Faridabad (IN)
(74) Representative: Bjerkén Hynell KB

(56) References cited:
- CN-A- 108 559 496
- CN-A- 111 944 585
- CN-A- 112 920 074
- CN-B- 108 690 609
- US-A1- 2015 361 334
- HE CHUANG ET AL: "Structural engineering design of carbon dots for lubrication", CHINESE CHEMICAL LETTERS, ELSEVIER, AMSTERDAM, NL, vol. 32, no. 9, 15 March 2021 (2021-03-15), pages 2693 - 2714, XP086842699, ISSN: 1001-8417, [retrieved on 20210315], DOI: 10.1016/J.CCLET.2021.03.026

## Description

### FIELD OF THE INVENTION

The present invention relates to a metal free, nontoxic, hydrocarbon fuel compatible fluorescent dispersion of carbon quantum dots (CQD) as marker as well as lubricity improver. More particularly, the present invention relates to process for preparation of the said hydrophobic carbon quantum dots, stability, and distinct fluorescence of those in different hydrocarbon mediums. A distinct fluorescence is used for determining adulteration of a hydrocarbon-based fuels.

### BACKGROUND OF THE INVENTION

Adulteration and counterfeiting are big issues in the oil and gas industry, for both producers and marketers. Some tagging agents or markers have been used to identify the mother product by specific instrument or technique in order to prevent adulteration and protect brand value. Several dye molecules, for example, a derivative of porphyrin molecules, are used as a fluorescent diesel marker (Dyes and Pigments 2009, 82, 26-30). Dyes made from cardanol and aniline derivatives that are used as gasoline and diesel markers and may be measured using UV-Vis spectroscopy (Ind. Eng. Chem. Res. 2004, 43, 4973-4978). By mass spectral analysis, a bisphenol derivatives dye is employed as a marker to identify liquid hydrocarbons, fuels, and oils (US9005315B2). Phthalocyanines and coumarin derivatives have been used as "silent" fluorescent markers because they are visually inconspicuous under ambient light, but fluorescence can easily be detected (US5525516; US5156653). However, their practical application is limited due to dye molecule solubility, photo bleaching characteristics, and detection by complex analytical techniques.

With the recent advancement and development of nanotechnology, it is now possible to solve the abovementioned challenges in the oil industry by using nanomaterials as a tagging agent. Because of their tunable luminous characteristics, superior quantification limits, and resistance to photo bleaching, fluorescent nanoparticles are being investigated for marking bulk liquids. CdSe quantum dots, are used to fluorescently tag diesel (Nascimento et.al, Fuel 2019, 239, 1055-1060).

Although CdSe quantum dots exhibit good fluorescence in diesel, these are not recommended due to their hazardous nature and the fact that metals are not permitted as per current regulations. Metal-free, nontoxic, fluorescent substance may be a suitable alternative for oil marker. Franco et.al., Ind. Eng. Chem. Res. 2020, 59, 25, 11359-11369 describes microwave-assisted synthesis of green, fluorescent carbon quantum dots and their application as an interwell tracer during crude production. These CQDs, on the other hand, are water dispersible rather than hydrophobic. Elis et al. (Saudi Aramco Company) (J. Petrol. Sci. Eng. 2017, 159, 443-450) investigated the use of CQDs as a tracer during water flood oil recovery. In a crosswell field test, Kosynkin et al. employed luminous carbogenic nanomaterials (World Petroleum Congress: Doha, Qatar, 2011). However, the majority of the carbon nanostructures discussed above are made in an aqueous media, which is incompatible with or less stable in a hydrophobic oil medium. Furthermore, the emission of blue or bluish green nanoparticles which are difficult to differentiate from most fuels and base oils. Photo luminescent carbon nanostructures (PCN) have recently been employed for bulk liquid tagging (US2020/0072753A1). These PCN are made in water and then put to a non-polar media as a suspended stock. Due to its limited solubility in tagged liquid, PCN can only be utilized up to a particular concentration. Despite its ability as a marker due to its fluorescence capabilities, poor dispersion stability in oils has been a practical difficulty. Chuang He et al., Chinese Chemical Letters, 2021, 32, 9, 2693-2714 describes several ways of preparing stable carbon dots, e.g. by reacting citric acid with oleyl amine.

Versatile materials are necessary as markers for bulk fuels, base oils and finished oils to limit inferior goods and counterfeiting. Traditional dye molecules lag due to their instability and photobleaching properties. Few carbon nanostructures are being considered as labels, but they are not suitable for such applications due to their low dispersion stability and the need for post-modification. In addition, the fluorescence from quantum dots largely overlaps with the emission from aromatic hydrocarbons present in various fuels and base oils. Thus, there is a need of stable hydrophobic metal free non-toxic material in non-polar hydrocarbon-based fuels and have distinct fluorescence as compared to base oils, formulated oils, and fuels.

### SUMMARY OF THE PRESENT INVENTION

Accordingly, the present invention relates to a one-step process for preparation of hydrophobic, fluorescent carbon quantum dots (CQDs), said process comprising the steps:
a. mixing a carbon source compound and a nitrogen containing nonpolar solvent with boiling point of above 175°C; and
b. autoclaving the solution of step (a), at a temperature of 180-220°C overnight, followed by cooling to room temperature, to obtain CQDs.

In another embodiment, of the present invention, the carbon source compound is selected from the group of citric acid, carbohydrate, amino acid, ascorbic acid, Phenyl diamine, cyclic π-bond containing organic molecules such as naphthoic acid, phthalic acid, dihydroxy biphenyl or mixture thereof.

In another embodiment, of the present invention, the solvent is selected from the group of oleyl amine, octyl amine, dodecyl amine, Octa decyl amine having boiling point above 175°C.

Further, as the carbon sources are carbonized to form CQDs at temperature above 180 °C, the functional group developed on surface of CQDs interacts with the solvent to stay dispersed. CQDs produced in a solvent with a boiling point exceeding 175 °C remains distributed. Solvents with boiling point below 175 °C requires a closed system, as the reaction is not possible in open system. As a result, in a closed system, the solvent maintains a gas-liquid equilibrium, and the material formed tends to precipitate.

In another preferred embodiment, the present invention includes no post modification processes such as washing, functionalizing, or redispersing. Hence, the amount of carbon sources carbonized at the process temperature remains dispersed in the system, ready for use. Thus, carbon loss due to the post treatment reaction process is avoided. In another embodiment, of the present invention, the hydrophobic, fluorescent carbon quantum dots are stable in non-polar hydrocarbon-based fuels/oils.

In another embodiment, the present invention relates to a method for improving the lubricity of non-polar hydrocarbon-based fuels by adding hydrophobic, fluorescent carbon quantum dots as prepared by the process defined above, to non-polar hydrocarbon-based fuel in amounts ranging from 10 to 200 ppm.

In another embodiment, of the present invention the CQDs are added to non-polar hydrocarbon-based fuels in amounts ranging from 10 to 200 ppm to improve lubricity.

In another embodiment, of the present invention, the one-step process for preparation of hydrophobic, fluorescent carbon quantum dots (CQDs), comprises the steps:
a. mixing ascorbic acid in oleyl amine as a non-polar solvent; and
b. autoclaving the solution of step (a), at a temperature of 200°C overnight, followed by cooling to room temperature, to obtain final dispersion of CQDs.

In another embodiment, the present invention provides a process for determining adulteration of a hydrocarbon-based fuels, said process comprising the steps:
a. adding CQDs to the fuel sample, to yield an additized fuel;
b. irradiating the additized fuel with light of specific wavelength and analyzing the emission.

In another embodiment, the additized fuel are irradiated with a light having wavelength in the range of 450-500 nm, and the CQDs additized fuel and fuel exhibit different color emission under irradiation.

In another embodiment, the present invention provides, that the CQDs are added in an amount of 2-200 ppm, preferably 2-100 pm; and CQDs additized fuel emits greenish yellow color.

In another embodiment, the present invention provides, that the hydrocarbon-based fuel is selected from the group of motor spirit, ethanol blended motor spirit, diesel, biodiesel, synthetic base oils, mineral base oils, or mixture thereof.

### OBJECTIVES OF THE PRESENT INVENTION

It is a primary objective of the invention to provide a process for preparation of stable hydrophobic carbon quantum dots (CQDs).

It is further objective of the present invention to provide a simple one step process for preparation of stable hydrophobic CQDs, and do not require any post-modification for compatibility with non-polar hydrocarbon matrix.

It is the further objective of the present invention to provide a highly stable CQDs, which are stable in non-polar hydrocarbon-based fuels (like motor spirits, ethanol blended motor spirits, diesel, biodiesel etc.) and in synthetic and mineral base oils (Gr I-Gr IV).

It is the further objective of the invention is to provide a CQDs having distinct fluorescence as compared to base oils, formulated oils, and fuels.

It is further objective of the present invention is to provide a stable hydrophobic CQDs which improves lubricity of the fuels.

Further objective of the present invention is to provide metal free, non-toxic, fluorescent CQD as a marker for non-polar hydrocarbon matrix.

### BRIEF DESCRIPTION OF DRAWINGS

Exemplary embodiments of the present disclosure are illustrated by way of example in the accompanying drawings.
**Figure 1****:** Illustrates (a) UV-Vis, (b) PL spectrum and (c) TEM image of as synthesized CQDs.
**Figure 2****:** Illustrates digital images of CQD dispersion in fuel (a) and oil (b). PL spectra and maximum fluorescence intensity of CQD dispersion in fuel (b,c) and oil (e,f) at different time points.
**Figure 3****:** Illustrates digital fluorescence images of CQD dispersion in BSVI diesel and motor spirit under 480 nm light.
**Figure 4****:** Illustrates digital fluorescence images of CQDs dispersion in Gr II & III base oil (a, b) and transmission oil (c) under 480 nm light.

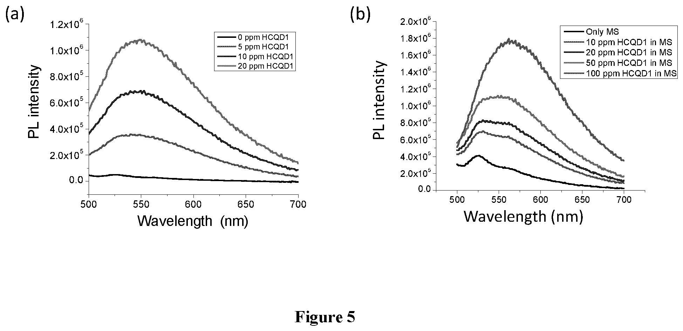

**Figure 5****:** Illustrates fluorescence spectrum of various concentration CQDs in diesel (a) and motor spirit (b).

### DETAILED DESCRIPTION OF THE INVENTION

For the purpose of promoting an understanding of the principles of the invention, reference will now be made to the embodiments in the specific language will be used to describe the same. It will nevertheless be understood that no limitation of the scope of the invention is thereby intended, such alterations and further modifications in the illustrated process, and such further applications of the principles of the invention as illustrated therein being contemplated as would normally occur to one skilled in the art to which the invention relates. Unless otherwise defined, all technical and scientific terms used herein have the same meaning as commonly understood by one of ordinary skilled in the art to which this invention belongs. The composition, process, and examples provided herein are illustrative only and not intended to be limiting.

The articles "a", "an" and "the" are used to refer to one or to more than one (i.e., to at least one) of the grammatical object of the article.

The terms "comprise" and "comprising" are used in the inclusive, open sense, meaning that additional elements may be included. It is not intended to be construed as "consists of only".

Throughout this specification, unless the context requires otherwise the word "comprise", and variations such as "comprises" and "comprising", will be understood to imply the inclusion of a stated element or step or group of element or steps but not the exclusion of any other element or step or group of element or steps.

The present invention provides a one step process for preparing oil dispersible highly fluorescent carbon quantum dots without any further modification.

In another aspect of the present invention, provides metal free, non-toxic, fluorescent CQD as a marker for non-polar hydrocarbon matrix.

In another embodiment, the present invention provides a process for preparation of color emitting hydrophobic CQDs by mixing of carbon source and a hydrophobic solvent, followed by solvothermal treatment of the mixture at temperature in a range of 180-220°C.

In another embodiment, the present invention, provides a greenish yellow color emitting hydrophobic CQDs, prepared by mixing ascorbic acid as a carbon source in an oleyl amine. Followed by transferring the solution into an autoclave and at a temperature of 200 °C. Reaction is continued overnight and cooled down to room temperature. Finally, obtained CQDs greenish yellow emission, is collected and stored for further characterization and use. It is to note that the reaction also can be carried out without an autoclave until unless the boiling point of the solvent used is higher than the reaction range of 180-220°C.

In another embodiment of the present invention, CQDs are prepared by mixing carbon source such as citric acid, carbohydrate, amino acid, cyclic π-bond containing organic molecules such as naphthoic acid, phthalic acid, dihydroxy biphenyl, and octyle amine, dodecyl amine, octadecyl amine as medium/solvent to carry out reaction. CQDs are not limited to greenish yellow emission. Other color emission CQDs are synthesized by changing the amount of carbon source, solvent, and temperature.

In another preferred embodiment of the present invention, CQDs are prepared by solvothermal treatment of the carbon source and nitrogen containing solvent at higher temperature of 180-220°C. CQDs are synthesized by changing the amount of carbon source, solvent, and temperature. Other carbon source such as citric acid, carbohydrate, amino acid, cyclic π-bond containing organic molecules can be used in synthesis of various color emitting hydrophobic CQDs.

In another embodiment, present invention provides compatible and dispersible CQDs, which are stable in various fuels and oils. CQDs in an amount of 1-1000 ppm is mixed with diesel, Motor spirit, base oils, formulated engine oil and/or transmission oil. The CQDs are highly stable in medium and show no agglomeration. As experimentally studied, even after 6 months, the fluorescence intensity remained steady, indicating stability.

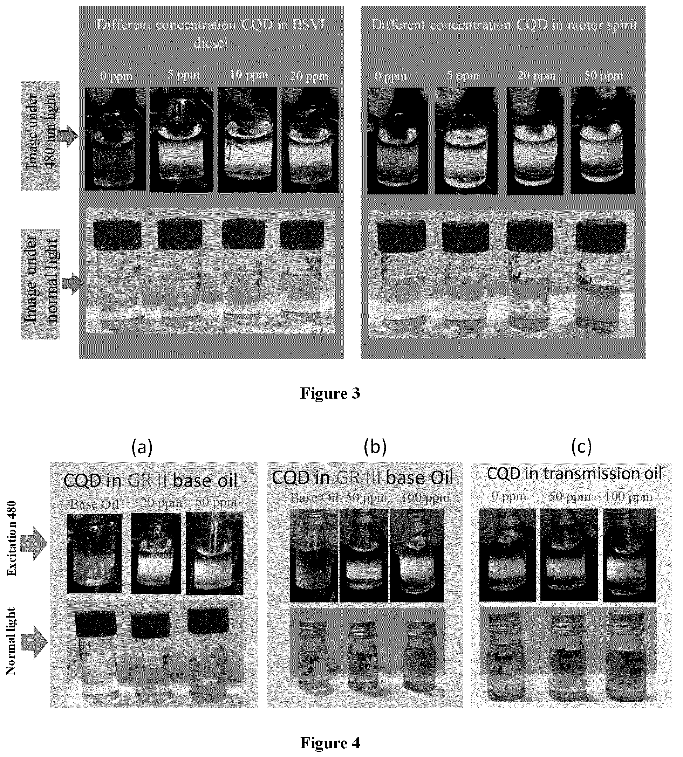

In another preferred embodiment of the present invention, CQDs are doped in fuel to identify the products using fluorescent properties of CQDs. Fuel containing 2-100 ppm CQDs, particularly 10-50 ppm, when irradiated with a 450-500 nm laser, emitted a characteristic greenish yellow light. In another embodiment, the present invention provides doping of CQD in base oils to identify the product using fluorescent properties of CQDs. By irradiating with 480 nm light, oils/fuels containing 2-100 ppm of CQD, especially 10-50 ppm, emits a characteristic greenish yellow emission. Under 480 nm light irradiation, specific transmission and engine oils containing CQD in the range of 2-100 ppm displays different emission and PL features as depicted in figure 3.

In another embodiment, the present invention provides that fluorescent intensity of CQD dispersion in fuels and oils varies with the amount of CQDs. Fluorescence intensity of CQD dispersed in fuel/oil increases as the amount of CQD increases.

In another embodiment, the present invention CQD improves lubricity of fuels/oils. Even without the use of a standard organic lubricity improver, the requisite lubricity is achieved when CQDs dispersed in diesel.

### EXAMPLES

The following examples are included herein for illustrative purposes only.

### Example 1:

### Synthesis of hydrophobic CQD

Hydrophobic fluorescent CQDs were synthesized via solvothermal method. The solvent for the synthesis is chosen carefully so that the CQDs are soluble or compatible to targeted non-polar hydrocarbons. In a typical reaction, 2-5 g ascorbic acid mixed with 70-100 mL oleyl amine. Next, the solution was transferred into an autoclave and temperature set at 200 °C. Reaction continued overnight and cooled down to room temperature. It is to note that the reaction also can be carried out without an autoclave until unless the boiling point of the solvent used is higher than the reaction range of 180-220°C. Finally, product collected and stored for further characterization and use. It is to note that other amines such as octyl amine, dodecyl amine and octadecyl amine can be used as solvent. Suitable carbon source such as citric acid, carbohydrate, amino acid, Phenyl diamine, other cyclic π-bond containing organic molecules such as naphthoic acid, phthalic acid, dihydroxy biphenyl etc., may be used for preparation of these hydrophobic CQDs.

### Example 2:

### Size, shape, and Optical properties of the hydrophobic CQD

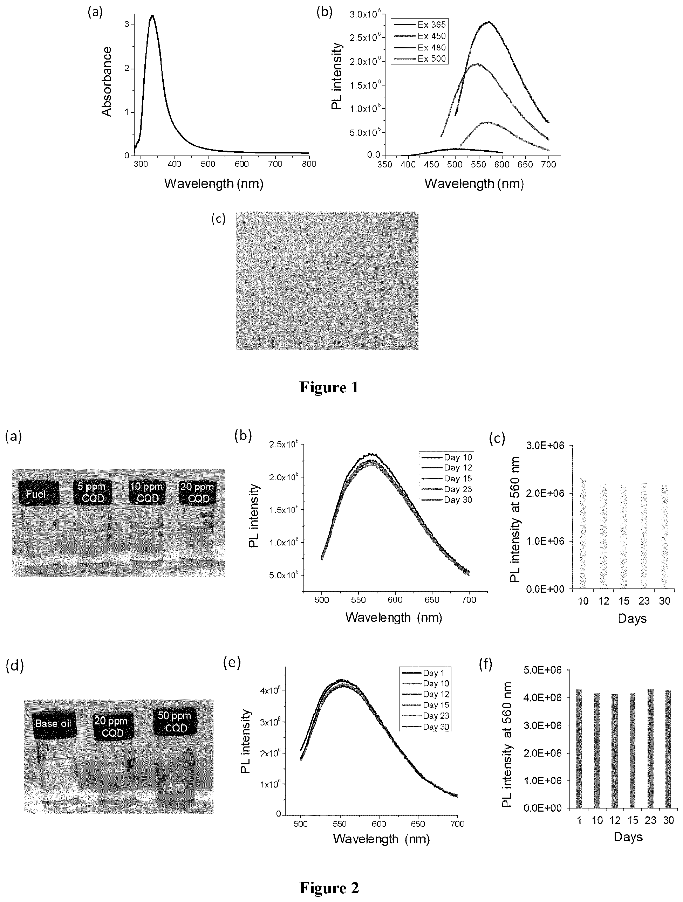

UV-Vis spectroscopy and Fluorescence spectrum of synthesized CQD diluted in hexane recorded to check absorption and emission properties of CQDs (Figure 1). Absorption spectrum of CQDs did not show any significant absorption peak in visible region although the solution becomes yellowish in color (Figure 1a). Photoluminescence (PL) spectrum of CQDs exhibit excitation dependent fluorescence and shows emission at 560 nm, when excited with 480 nm. (Figure 1b). Such excitation dependent fluorescence property is common for CQDs nanoparticles and is dependent on the presence of various energy level associated with defect of hetero atom N and O in the nanoparticles. TEM microscopy technique was used to check the size and diversity of as synthesized CQD nanoparticles. TEM image of as synthesized CQD clearly shows formation of monodispersed spherical shape nanoparticles of size around 2-7 nm (Figure 1c).

### Example 3:

### Dispersion stability of CQD in hydrocarbon matrices

Dispersion stability of CQDs in fuels and oils accessed by observing presence of any agglomeration and change in fluorescence of solution at different time point. In the digital images of CQDs in fuel (Figure 2a) and oil (Figure 2b) did not show any agglomeration in solution. Corresponding fluorescence spectrum and intensity of fluorescence of CQDs dispersion solution in fuels (Figure 2b-c) and oil (Figure 2e-f) showed that both the spectral pattern and intensity of fluorescence remain unchanged over several days' time. Thus, these results prove that CQDs in fuel and oil have high dispersion stability

### Example 4:

### Fluorescence imaging of CQD doped fuels

Various concentrations of CQD were added to various fuels to test the capacity of CQDs to identify the product using their fluorescence qualities. Next, 480 nm light was irradiated on CQD additized fuel solution and color of the solutions observed. Digital images of fluorescence of CQD dispersion in BS VI diesel & motor spirit fuel showed distinct greenish yellow color compared to solution without CQD. (Figure 3).

### Example 5:

### Fluorescence imaging of CQD doped oils

To evaluate the feasibility of CQD fluorescence properties to identify oil, different amounts of CQDs were added to different base oils and created oils. Next, CQDs additized oils were kept under 480 nm light and emission color of CQDs in various solution examined. Digital fluorescence images of CQD additized base oil showed distinct yellowish emission compared to only base oils (Figure 4 a, b). CQD additized transmission oil also exhibit significant greenish yellow emission under irradiation of 480 nm light (Figure 4c).

### Example 6:

### Effect of concentration of CQD in matrix on intensity

Changes in fluorescence with the amount of CQD present in solution investigated by taking fluorescence spectrum of various concentration of CQD in fuels. The fluorescence spectrum of various concentration CQD in BSVI diesel and motor spirit presented in Figure 4. The results showed that fluorescence intensity decreases with decreasing the concentration of CQD in both fuels. This indicated that's present invention CQD protects the mother product from the mixing with other cheap product.

### Example 7:

### Lubricity study of fuel doped with CQD

CQD doped fuel prepared by adding different amount of CQDs stock solution and lubricity of the samples vis a vis base fuel was evaluated using HFRR technique. The outcome of the HFRR results of diesel is represented in table 1. Wear scar diameter (WSD) of the neat diesel without any lubricity improver or CQD is 590, which notably reduces to 411 microns after addition of CQD of concentration 30. The same value for commercial diesel (BS VI) containing organic lubricating additive is 425. Hence, the CQDs also works as lubricity improver in diesel fuel. We also studied effect of CQD on commercial diesel, which normally contains lubricity improver to compensate the lost lubricity with sulfur removal. With addition of 20 and 50 ppm of CQD, the WSD value decreases to 388 and 386 microns compare to WSD value of diesel (425 microns). Hence, the CQDs function in synergetic with the lubricity improver without any negative interaction. All these data demonstrated that CQD can be used as lubricating additive for diesel and also work in synergetic with lubricating additive for marker purpose.

**Table 1- HFRR results of CQDs dispersion in diesel**

| **HFRR Test** | **Matrix** | **Lubricity Improver** | **Amount of CQD in ppm** | **WSD value in micron** |
|---|---|---|---|---|
| 1. | Diesel (BS VI) | No | 0 | 590 |
| 2. | Diesel (BS VI) | No | 30 | 411 |
| 3. | Diesel (BS VI) | No | 60 | 405 |
| 4. | Diesel (BS VI) | Yes | 0 | 425 |
| 5. | Diesel (BS VI) | Yes | 20 | 388 |
| 6. | Diesel (BS VI) | Yes | 50 | 386 |

### Advantage of present invention:

1. Highly stable CQDs in non-polar hydrocarbon-based fuels (motor spirit, ethanol blended motor spirit, diesel, biodiesel etc.) and both in synthetic and mineral base oils (Gr I-Gr IV).
2. CQDs have distinct Fluorescence as compared to base oils and fuels.
3. CQDs as a lubricity improver in hydrocarbon-based fuels.

## Claims

1. A one-step process for preparation of hydrophobic, fluorescent carbon quantum dots (CQDs), said process comprising the steps:
a. mixing a carbon source compound and a nitrogen containing non-polar solvent with boiling point of above 175°C; and
b. autoclaving the solution of step (a), at a temperature of 180-220°C overnight, followed by cooling to room temperature, to obtain CQDs.

2. The process according to claim 1, wherein the carbon source compound is selected from the group of citric acid, carbohydrate, amino acid, ascorbic acid, Phenyl diamine, cyclic π-bond containing organic molecules such as naphthoic acid, phthalic acid, dihydroxy biphenyl, or mixture thereof.

3. The process according to any of the previous claims, wherein the nitrogen containing non-polar solvent is selected from the group of oleyl amine, octyl amine, dodecyl amine, Octa decyl amine having boiling point above 175°C.

4. A method for improving the lubricity of non-polar hydrocarbon-based fuels by adding hydrophobic, fluorescent carbon quantum dots as prepared by the process according to any of claims 1-3 to non-polar hydrocarbon-based fuel in amounts ranging from 10 to 200 ppm.

5. A one-step process for preparation of hydrophobic, fluorescent carbon quantum dots (CQDs) as claimed in claim 1, comprising the steps:
a. mixing ascorbic acid and oleyl amine as a non-polar solvent; and
b. autoclaving the solution of step (a), at a temperature of 200°C overnight, followed by cooling to room temperature, to obtain CQDs.

6. A process for determining adulteration of a non-polar hydrocarbon-based fuel, said process comprising the steps:
a. adding CQDs as prepared by the process according to claims 1-3 and 5 to the fuel sample, to yield an additized fuel;
b. irradiating the additized fuel with light of a specific wavelength.

7. The process according to claim 6, wherein the non-polar hydrocarbon-based fuel is selected from the group of motor spirit, ethanol blended motor spirit, diesel, biodiesel, or mixture thereof.

8. The process according to claim 6, wherein the additized fuel is irradiated with a light having wavelength in the range of 450-500 nm.

9. The process according to claim 6, wherein the CQDs additized fuel and the non-polar hydrocarbon-based fuel exhibit different color emissions under irradiation.

10. The process according to claim 6, wherein the CQDs are added in an amount of 2-200 ppm, preferably 2-100 pm; and the CQDs additized fuel emits greenish yellow color.

## Patentansprüche

1. Einstufiger Prozess zur Herstellung von hydrophoben, fluoreszierenden Kohlenstoffquantenpunkten (CQDs), der Prozess umfassend die Schritte:
a) Mischen einer Kohlenstoffquelle und eines stickstoffhaltigen unpolaren Lösungsmittels mit einem Siedepunkt von über 175 °C; und
b) Autoklavieren der Lösung aus Schritt (a) bei einer Temperatur von 180-220 °C über Nacht, gefolgt von einem Abkühlen auf Raumtemperatur, um CQDs zu erhalten.

2. Prozess nach Anspruch 1, wobei die Kohlenstoffquelle aus der Gruppe Zitronensäure, Kohlenhydrat, Aminosäure, Ascorbinsäure, Phenyldiamin, zyklische π-Bindungen enthaltende organische Moleküle wie Naphthoesäure, Phthalsäure, Dihydroxybiphenyl oder Mischungen davon ausgewählt ist.

3. Prozess nach einem der vorstehenden Ansprüche, wobei das stickstoffhaltige unpolare Lösungsmittel aus der Gruppe Oleylamin, Octylamin, Dodecylamin, OctaDecylamin mit einem Siedepunkt über 175 °C ausgewählt ist.

4. Verfahren zur Verbesserung der Schmierfähigkeit von unpolaren Kraftstoffen auf Kohlenwasserstoffbasis durch Zugabe von hydrophoben, fluoreszierenden Kohlenstoffquantenpunkten, wie sie nach dem Verfahren nach einem der Ansprüche 1 bis 3 hergestellt werden, zu unpolarem Kraftstoff auf Kohlenwasserstoffbasis in Mengen von 10 bis 200 ppm.

5. Einstufiger Prozess zur Herstellung von hydrophoben, fluoreszierenden Kohlenstoffquantenpunkten (CQDs) nach Anspruch 1, umfassend die Schritte:
a) Mischen von Ascorbinsäure und Oleylamin als unpolares Lösungsmittel; und
b) Autoklavieren der Lösung aus Schritt (a) bei einer Temperatur von 200 °C über Nacht, gefolgt von einem Abkühlen auf Raumtemperatur, um CQDs zu erhalten.

6. Prozess zum Bestimmen der Verfälschung eines unpolaren Kraftstoffs auf Kohlenwasserstoffbasis, der Prozess umfassend die Schritte:
a) Hinzufügen von CQDs, wie sie nach dem Prozess nach den Ansprüchen 1 bis 3 und 5 hergestellt wurden, zu der Kraftstoffprobe, um einen additivierten Kraftstoff zu erhalten;
b) Bestrahlen des additierten Brennstoffs mit Licht einer bestimmten Wellenlänge.

7. Prozess nach Anspruch 6, wobei der unpolare Kraftstoff auf Kohlenwasserstoffbasis aus der Gruppe von Testbenzin, mit Ethanol gemischtem Testbenzin, Diesel, Biodiesel oder Mischungen davon ausgewählt ist.

8. Prozess nach Anspruch 6, wobei der additivierte Brennstoff mit einem Licht mit einer Wellenlänge im Bereich von 450-500 nm bestrahlt wird.

9. Prozess nach Anspruch 6, wobei der mit CQDs additivierte Brennstoff und der unpolare Brennstoff auf Kohlenwasserstoffbasis bei Bestrahlung unterschiedliche Farbemissionen aufweisen.

10. Prozess nach Anspruch 6, wobei die CQDs in einer Menge von 2-200 ppm, vorzugsweise 2-100 pm, zugesetzt werden; und der mit CQDs versetzte Kraftstoff eine grünlich-gelbe Farbe aufweist.

## Revendications

1. Procédé en une seule étape pour la préparation de points quantiques de carbone (CQD) fluorescents et hydrophobes, ledit procédé comprenant les étapes suivantes:
a. mélanger un composé source de carbone et un solvant non polaire contenant de l'azote avec un point d'ébullition supérieur à 175 °C ; et
b. autoclaver de la solution de l'étape (a), à une température de 180-220°C pendant la nuit, suivi d'un refroidissement à température ambiante, pour obtenir des CQD.

2. Procédé selon la revendication 1, dans lequel le composé source de carbone est sélectionné parmi ceux du groupe constitué par l'acide citrique, les glucides, les acides aminés, l'acide ascorbique, la phényldiamine, les molécules organiques contenant une liaison π - cyclique telles que l'acide naphtoïque, l'acide phtalique, le dihydroxybiphényle ou un mélange de ceux-ci.

3. Procédé selon l'une quelconque des revendications précédentes, dans lequel le solvant non polaire contenant de l'azote est sélectionné parmi ceux du groupe constitué par l'oléylamine, l'octylamine, la dodécylamine, l'octadécylamine ayant un point d'ébullition supérieur à 175°C.

4. Procédé pour améliorer la lubrification de carburants à base d'hydrocarbures non polaires en ajoutant des points quantiques de carbone fluorescents hydrophobes tels que préparés par le procédé selon l'une quelconque des revendications 1 à 3 à un carburant à base d'hydrocarbures non polaires dans des quantités allant de 10 à 200 ppm.

5. Procédé en une seule étape pour la préparation de points quantiques de carbone (CQD) fluorescents et hydrophobes selon la revendication 1, comprenant les étapes:
a.mélanger de l'acide ascorbique et de l'oléylamine comme solvant non polaire ; et
b. autoclaver de la solution de l'étape (a), à une température de 200°C pendant la nuit, suivi d'un refroidissement à température ambiante, pour obtenir des CQD.

6. Procédé de détermination de l'adultération d'un carburant à base d'hydrocarbures non polaires, ledit procédé comprenant les étapes:
a. ajouter des CQD préparés par le procédé selon les revendications 1-3 si 5 à l'échantillon de carburant, pour produire un carburant additivé ;
b. irradier le carburant additivé avec une lumière d'une longueur d'onde spécifique.

7. Procédé selon la revendication 6, dans lequel le carburant à base d'hydrocarbures non polaires est sélectionné parmi ceux du groupe constitué par l'essence pour moteur, l'essence pour moteur mélangée à de l'éthanol, le diesel, le biodiesel ou un mélange de ceux-ci.

8. Procédé selon la revendication 6, dans lequel le carburant additivé est irradié avec une lumière ayant une longueur d'onde dans la plage de 450 à 500 nm.

9. Procédé selon la revendication 6, dans lequel le carburant additivé en CQD et le carburant non polaireà base d'hydrocarbures présentent des émissions de couleurs différentes sous irradiation.

10. Procédé selon la revendication 6, dans lequel les CQD sont ajoutés en une quantité de 2 à 200 ppm, de préférence de 2 à 100 pm ; et le carburant additivé en CQD émet une couleur jaune verdâtre.
